# EUROPÄISCHE PATENTSCHRIFT

(11) **EP 2 569 271 B1**
(45) Veröffentlichungstag und Bekanntmachung des Hinweises auf die Patenterteilung: **14.05.2014**
(21) Anmeldenummer: 11718124.8
(22) Anmeldetag: 10.05.2011
(51) Int. Cl.: C07C 29/141, C07C 31/20

(54) **VERFAHREN ZUR HERSTELLUNG VON NEOPENTYLGLYKOL**
PROCESS FOR PREPARING NEOPENTYL GLYCOL
PROCÉDÉ DE PRODUCTION DE NÉOPENTYLGLYCOL

(30) Priorität: 12.05.2010 EP 10162619
(43) Veröffentlichungstag der Anmeldung: 20.03.2013
(73) Patentinhaber: BASF SE, 67056 Ludwigshafen (DE)
(72) Erfinder: STEINIGER, Michael, 67435 Neustadt (DE); GUIXA GUARDIA, Maria, 68163 Mannheim (DE); STEHMEIER, Kai, 68199 Mannheim (DE)
(86) Internationale Anmeldenummer: PCT/EP2011/057538
(87) Internationale Veröffentlichungsnummer: WO 2011/141470

(56) Entgegenhaltungen:
- WO-A1-01/51438
- WO-A1-98/29374
- WO-A1-2007/099064
- WO-A2-2010/000382

## Beschreibung

Die vorliegende Erfindung betrifft ein Verfahren zur Herstellung von Neopentylglykol (NPG) durch Hydrierung von Hydroxypivalinaldehyd (HPA).

Neopentylglykol wird als Rohstoff für die Herstellung gesättigter Polyesterharze für Pulverlacke sowie für glasfaserverstärkte Kunststoffe eingesetzt.

Die Herstellung von Neopentylglykol erfolgt in der Regel in einem zweistufigen Verfahren, bei dem zunächst Isobutyraldehyd (IBA) mit Formaldehyd (FA) in einer Aldoladdition zu Hydroxypivalinaldehyd umgesetzt wird, welches in einer zweiten Verfahrensstufe direkt zu NPG hydriert werden kann.

In der ersten Verfahrensstufe (Aldolreaktion) wird im Allgemeinen Isobutyraldehyd in einer Aldolreaktion mit Formaldehyd in Gegenwart von tertiären Aminen als Katalysator umgesetzt.
Der Austrag aus der Aldolreaktion enthält üblicherweise HPA sowie nicht umgesetzte Ausgangsverbindungen, wie Formaldehyd, IBA, sowie den eingesetzten tertiären Aminkatalysator und Wasser.
Üblicherweise enthält der Austrag auch Verunreinigungen und Nebenprodukte aus der Aldolreaktion, wie Ameisensäure, die durch Cannizzaro- oder Tischtschenko-Reaktion aus Formaldehyd entstehen können, und Formiat-Salze der eingesetzten Aminkatalysatoren, wie Trimethylammoniumformiat.
Nach der Aldolisierung werden in der Regel nicht umgesetzte Aldehyde und ein Teil der Amin-Base destillativ abgetrennt und in die Aldolreaktion zurückgeführt.
Im Destillationssumpf zurück bleiben üblicherweise HPA, Wasser, Trialkylammoniumformiat und Ameisensäure. Ameisensäure wird im Allgemeinen bei der Aldolisierung als Nebenprodukt in einer Cannizzaro-Reaktion aus Formaldehyd gebildet. Der Destillationssumpf weist daher in der Regel einen sauren pH-Wert auf (pH < 7).
In der Regel wird der Austrag aus dem Kolonnensumpf als zu hydrierendes Gemisch in einen Hydrierreaktor geleitet und an einem heterogenen Katalysator in Gegenwart von Wasserstoff zu NPG hydriert.

Um die Bildung von Nebenprodukten zu reduzieren und um die Standzeit des Katalysators zu verlängern und eine schnelle Aktivitätsabnahme des Katalysators zu vermeiden, wird im Stand der Technik gelehrt, dass der pH-Wert des zu hydrierenden Gemisches aus der Aldolisierung auf einen bestimmten pH-Bereich eingestellt werden soll.

So wird in der WO-A-2004092097 ein Hydrierverfahren offenbart, bei dem durch Zusatz eines tertiären Amins zum zu hydrierenden Gemisch aus der Aldolisierung ein pH-Wert in diesem Gemisch von 6,3 bis 7,8 eingestellt wird.

In der WO-A-2007099064 wird zudem offenbart, dass während der Hydrierreaktion eine Vielzahl von Nebenreaktionen stattfinden, die den pH-Wert im Hydrierreaktor verändern können.
So wird beispielsweise Ameisensäure, die bei der Aldolisierung als Nebenprodukt über eine Cannizzaro-Reaktion aus Formaldehyd gebildet wird, während der Hydrierung katalytisch zu CO₂ und H₂ bzw. zu CO und H₂O zersetzt. Die Zersetzungsrate des unerwünschten Nebenprodukts Ameisensäure hängt neben der Temperatur entscheidend vom Alter des Katalysators ab. Mit zunehmendem Alter des Katalysators nimmt auch die Zersetzungsrate von Ameisensäure unter konstanten Reaktionsbedingungen permanent ab.
Daher ist der pH-Wert im Reaktor nicht mit dem pH-Wert des Hydrierzulaufs aus der Aldolisierung korreliert. Die Differenz der pH-Werte zwischen Zulauf (Feed) und Austrag wird durch eine Vielzahl von nicht berechenbaren Faktoren beeinflusst, wie der Aktivität des Katalysators bezüglich der Zersetzung von Ameisensäure, sowie durch Temperatur, Abgasmenge und der Katalysatorbelastung.
Die WO-A-2007099064 lehrt weiterhin, dass das Ausmaß und die Geschwindigkeit verschiedener Nebenreaktionen, die während der Hydrierung stattfinden, abhängig vom pH-Wert im Hydrierreaktor sind.
So nimmt beispielsweise bei hohen pH-Werten die Retro-Aldol-Reaktion und die Tischtschenko-Reaktion stark zu.
Bei der Retro-Aldol-Reaktion werden im Allgemeinen die Methylolalkanale in die entsprechenden Ausgangsaldehyde gespalten, die zu unerwünschten Nebenprodukten hydriert werden (bei der NPG-Herstellung entstehen so Isobutanol und Methanol). Dadurch wird die Ausbeute bei der Hydrierung entsprechend vermindert.
Die Tischtschenko-Reaktion von Methylolalkanalen führt zu der Bildung der entsprechenden Methylolalkansäuremethylolestern. So wird beispielsweise Hydroxypivalinsäureneopentylglykolester (HPN)aus Hydroxypivalinaldehyd (HPA) gebildet, welches teilweise zu NPG und Hydroxypivalinsäure (HPS) hydrolysiert, was wiederum zur Absenkung des pH-Wertes und der Selektivität führt.
Weiterhin können sich während der Hydrierung Acetale bilden. Im Falle der NPG-Synthese wird bei erhöhter Temperatur auch die Bildung des cyclischen Acetals von NPG und Hydroxypivalinaldehyd (HPA) verstärkt beobachtet. Dieses Nebenprodukt ist destillativ nicht von NPG zu trennen und führt daher zu einem unreineren Wertprodukt. Auch die Acetalbildung ist pH-Wert abhängig.
Zur Verringerung von Nebenreaktionen und zur Verbesserung der Selektivität bei der Hydrierung lehrt WO-A-2007099064 die Einstellung eines pH-Wertes von 7,0 bis 9,0 im Hydrieraustrag. Die Einstellung des pH-Wertes im Hydrieraustrag erfolgt durch Zugabe mindestens eines tertiären Amins, einer anorganischen Base, einer anorganischen Säure oder organischen Säure zum zu hydrierenden Gemisch aus der Aldolisierung.

Die JP-A-2004182622 beschreibt ein Hydrierverfahren bei dem der pH-Wert im zu hydrierenden Gemisch aus der Aldolisierung auf einen pH von 5,5 - 7,5 eingestellt wird, um ein Austragen des Aktivmetalls aus dem Katalysator bei kleineren pH-Werten zu vermindern, da die Austragung von Aktivmetall zu einem kontinuierlichen Aktivitätsverlust des Katalysators führt und Metallspuren in der weiteren Aufarbeitung stören. Bei höheren pH-Werten wurden Aldolkondensationen beobachtet, die die Selektivität des Verfahrens verringern.

Im Stand der Technik wird beschrieben, dass die Zugabe von pH-Regulatoren, z.B. tertiäre Amine, anorganische Base, anorganische Säure oder organische Säure, zum zu hydrierenden Gemisch aus der Aldolisierung erfolgen soll, um den pH-Wert im Hydrierreaktor zu regulieren.
Nähere technische Einzelheiten zur Dosierung, insbesondere bezüglich der genauen Zusammensetzung des zu hydrierenden Gemischs aus der Aldolisierung vor und nach der Dosierung des pH-Regulators oder der genauen Zulaufstelle, werden in den genannten Offenbarungen nicht explizit beschrieben.

Im Rahmen der vorliegenden Erfindung wurde nun gefunden, dass die Hydrierung von HPA weiter verbessert werden kann, wenn man einen HPA-enthaltenden Strom vor der Einleitung in den Hydrierreaktor mit einem NPG-enthaltenden Strom zusammenführt, und wenn man die Bedingungen der Zusammenführung dieser Ströme und die Bedingungen der Zuführung des pH-Regulators in geeigneter Weise miteinander kombiniert.

Die Aufgabe der vorliegenden Erfindung bestand darin, ein verbessertes Verfahren zur Hydrierung von HPA zur Verfügung zu stellen, in dem Nebenreaktionen zurückgedrängt und sowohl die Ausbeute als auch die Selektivität gegenüber den Hydrierungen gemäß dem Stand der Technik weiter verbessert werden sollten. Außerdem sollte die Standzeit der in die Hydrierung eingesetzten Katalysatoren weiter erhöht werden. Zudem sollte die zugegebene Menge der pH-Regulatoren verringert werden, um die Menge der eingesetzten Rohstoffe zu reduzieren.

Die Aufgabe der vorliegenden Erfindung wurde gelöst durch ein
Verfahren zur Herstellung Neopentylglykol (NPG) durch kontinuierliche Hydrierung von Hydroxypivalinaldehyd (HPA)
mit Wasserstoff,
in der Flüssigphase,
in Gegenwart eines Hydrierkatalysators,
in einem Hydrierreaktor (5),
wobei man einen HPA-enthaltenden Strom (1) mit einem NPG-enthaltenden Strom (2) zu einem Hydrierfeed (4) zusammenführt und man den Hydrierfeed (4) in den Hydrierreaktor (5) einleitet
und man zusätzlich mindenstens einen pH-Regulator (3) ausgewählt aus der Gruppe bestehend aus tertiärem Amin, einer anorganischen Base, einer anorgani-schen und einer organischen Säure, dem HPA-enthaltenden Strom (1) oder dem NPG-enthaltenden Strom (2) oder dem Hydrierfeed (4) zuführt,
um einen pH-Wert von 7,0 bis 9,0 am Ausgang des Hydrierreaktors einzustellen, dadurch gekennzeichnet, dass
das Gewichtsverhältnis von HPA zu NPG im Hydrierfeed (4) im Bereich von 1:100 bis 50:100 liegt und
der Anteil von HPA und NPG im Hydrierfeed (4) mindestens 50 Gew.-% beträgt, bezogen auf den Hydrierfeed,
wobei für den Fall, dass der pH-Regulator (3) dem HPA-enthaltenden Strom (1) zugeführt wird entweder
der HPA-enthaltende Strom (1) weniger als 50 Gew.-% HPA enthält oder die Verweilzeit zwischen der Zuführung des pH-Regulators (3) und der Zusammenführung des NPG-enthaltenden Stroms (2) mit dem HPA-enthaltenden Strom (1) weniger als 5 Minuten beträgt oder
die Temperatur des HPA-enthaltenden Stroms (1) weniger als 75°C beträgt.

In dem erfindungsgemäßen Verfahren wird ein HPA-enthaltender Strom und ein NPG-enthaltender Strom zusammengeführt. Die zusammengeführten Ströme werden im Rahmen der vorliegenden Erfindung als "Hydrierfeed" bezeichnet. Der Begriff Hydrierfeed bezeichnet demgemäß den Strom, der aus der Zusammenführung des HPA-enthaltenden Stroms mit dem NPG-enthaltenden Stroms resultiert, und der in den Hydrierreaktor eingeleitet wird.

In das erfindungsgemäße Verfahren wird ein HPA-enthaltender Strom eingesetzt.

Der eingesetzte HPA-enthaltende Strom ist bevorzugt ein Reaktionsaustrag aus der Aldolreaktion von Isobutyraldehyd und Formaldehyd.
Ein solcher Reaktionsaustrag kann beispielsweise gemäß der Offenbarung der WO-A-98/28253 oder der DE-A-1957591 durch Umsetzung von IBA mit Formaldehyd hergestellt werden. Es wird dabei in der Regel so verfahren, dass der in die Aldolreaktion eingesetzte IBA mit der 1- bis 8-fachen Mengen Formaldehyd in Gegenwart eines tertiären Amins (Aldolisierung) umgesetzt wird.
Als tertiäre Amine können Amine, wie sie z. B. in DE-A 28 13 201 und DE A 27 02 582 beschrieben sind, eingesetzt werden. Besonders bevorzugt sind Tri-n-alkylamine, insbesondere Triethylamin, Tri-n-propylamin, Tri-n-butylamin und Trimethylamin.
Ganz besonders bevorzugt sind Trimethylamin (TMA), Triethylamin (TEA) und Tri-n-propylamin (TPA), da diese Verbindungen in der Regel einen niedrigeren Siedepunkt als die bevorzugt gebildeten Polymethylole aufweisen und somit die destillative Entfernung aus dem Reaktionsgemisch erleichtert wird. Insbesondere bevorzugt wird Trimethylamin (TMA) als tertiäres Amin in die Aldolisierung eingesetzt.

Das Umsetzungsprodukt wird nachfolgend üblicherweise destillativ aufgetrennt. Hierbei wird der Austrag aus der Aldolreaktion einer Destillationsvorrichtung, im Allgemeinen einer Kolonne, zugeführt, in der er in leichter und schwerer flüchtige Bestandteile aufgetrennt wird.
Dabei werden die Destillationsbedingungen in der Regel so gewählt, dass sich eine Fraktion aus Leichtsiedern bildet, in der als wesentliche Komponenten nicht umgesetztes Alkanal und gegebenenfalls Wasser, Formaldehyd und Methanol enthalten sind. Bei der Verwendung von Trimethylamin (TMA) als tertiäres Amin, werden die Destillationsbedingungen so gewählt, dass TMA auch zum Teil in der in Leichtsiederfraktion enthalten ist und zum geringen Teil im Sumpfprodukt vorhanden ist. Bei der Verwendung von Triethylamin (TEA), werden die Destillationsbedingungen so gewählt, dass TEA im Sumpfprodukt angereichert werden.
Diese sogenannte Leichtsiederfraktion kann in die erste Stufe des Hydrierverfahrens, der Aldolreaktion, zurückgeführt werden oder einer weiteren Aufarbeitungsstufe zugeführt werden.
Nach Abtrennung der Leichtsiederfraktion verbleibt nach der geschilderten destillativen Aufarbeitung ein schwerer flüchtiges Sumpfprodukt, das im Wesentlichen aus HPA, Wasser, Ameisensäure sowie Amin-Formiat besteht, das als HPA-enthaltender Strom in das erfindungsgemäße Verfahren eingesetzt werden kann.

Es ist jedoch auch möglich einen HPA-enthaltenden Strom einzusetzen, der nach anderen Verfahren des Standes der Technik, beispielsweise nach den aus WO 01/51438, WO 97/17313 und WO 98/29374 bekannten Verfahren hergestellt wurde.

Der Gehalt an HPA im Austrag der Aldolreaktion nach Abtrennung der Leichtsiederfraktion in einem üblichen HPA-enthaltendem Strom liegt gemäß den voranstehend genannten Offenbarungen in einem breiten Bereich von 20 bis 95 Gew.-%, bevorzugt von 40 bis 85 Gew.% und besonders bevorzugt von 50 bis 80 Gew.-%.

Der HPA-enthaltende Strom aus der Aldolisierung enthält neben HPA in der Regel zusätzlich Wasser sowie weitere, sonstige organische Verbindungen, beispielsweise nicht umgesetzte Edukte oder Nebenprodukte der Aldolisierung. Beispiele für sonstige organische Verbindungen sind Acetale, Halbacetale, Methanol, Ester, Aminformiat etc.. Das Wasser wird dem Reaktionssystem in der Regel über die Dosierung des Formaldehyds zugeführt, da Formaldehyd im Allgemeinen als wässrige Lösung eingesetzt wird.
Bevorzugt enthält der HPA-enthaltende Strom aus der Aldolisierung, der in das erfindungsgemäße Verfahren eingesetzt wird, weniger als 10 Gew.-% NPG, besonders bevorzugt weniger als 5 Gew.-% NPG und insbesondere bevorzugt weniger als 3 Gew.-% NPG, bezogen auf den HPA-enthaltenden Strom.
In einer bevorzugten Ausführungsform enthält der Austrag der Aldolreaktion nach Abtrennung der Leichtsiederfraktion kein zusätzliches organisches Lösungsmittel, um die Konzentration vom HPA in HPA-enthaltenden Strom nicht zu verdünnen. Eine hohe Konzentration von HPA im Hydrierfeed ermöglicht nämlich eine geringere Dimensionierung des Hydrierreaktors und den Einsatz geringerer Katalysatormengen, wodurch insgesamt die Investitions- und Betriebskosten verringert werden können.

Bevorzugt beträgt die Zusammensetzung des HPA-enthaltenden Stroms aus der Aldolisierung nach Abtrennung der Leichtsiederfraktion, der in das erfindungsgemäße Verfahren eingesetzt wird:
50 bis 85 Gew.-% HPA;
15 bis 50 Gew.-% Wasser;
Rest: sonstige organische Verbindungen.
und besonders bevorzugt
60 bis 80 Gew.-% HPA;
20 bis 40 Gew.-% Wasser;
Rest: sonstige organische Verbindungen.

Wie nachfolgend ausgeführt, kann auch ein HPA-enthaltender Strom eingesetzt werden, der weniger als 50 Gew.-% HPA enthält. Wie nachfolgend erläutert, wird ein solcher HPA-enthaltender Strom bevorzugt dann eingesetzt, wenn die Zuführung des pH-Regulators zu dem HPA-enthaltenden Strom erfolgt.

In dem erfindungsgemäßen Verfahren wird weiterhin ein Strom zugeführt, der NPG enthält. Dieser Strom wird im Rahmen der vorliegenden Erfindung auch als NPG-enthaltender Strom bezeichnet.

Der NPG-enthaltende Strom enthält bevorzugt mehr als 30 Gew.-% NPG, besonders bevorzugt 40 bis 95 Gew.-%, ganz besonders bevorzugt 50 bis 90 Gew.-% und insbesondere bevorzugt 60 bis 80 Gew.-%.

Üblicherweise enthält der NPG-enthaltende Strom neben NPG zusätzlich Wasser, sowie weitere sonstige organische Verbindungen enthalten, beispielsweise nicht umgesetzte Edukte oder Nebenprodukte der Aldolisierung bzw. deren Hydrierungsprodukte. Beispiele für sonstige organische Verbindungen sind Acetale, Halbacetale, Methanol, Ester, Aminformiat etc..

In einer ganz besonderen Ausführungsform ist NPG-enthaltende Strom ein Teilstrom des Austrags aus dem Hydrierreaktor.

Dieser Teilstrom enthält bevorzugt
10 bis 50 Gew.-% Wasser,
50 bis 90 Gew.-% NPG,
Rest: sonstige organische Verbindungen,
und ganz besonders bevorzugt
20 bis 40 Gew.-% Wasser,
60 bis 80 Gew.-% NPG,
Rest: sonstige organische Verbindungen.

Als NPG-enthaltender Strom kann aber auch ein Strom eingesetzt werden, der aufgereinigtes NPG und vorzugsweise Wasser enthält, beispielsweise kann NPG eingesetzt werden, das in ein oder mehreren Stufen destillativ aufgereinigt wurde.

Erfindungsgemäß werden der HPA-enthaltende Strom und der NPG-enthaltende Strom zu einem Hydrierfeed zusammengeführt.

Die jeweiligen Zufuhrmengen werden so eingestellt, dass das Gewichtsverhältnis von HPA zu NPG im Hydrierfeed im Bereich von 1:100 bis 50:100, bevorzugt im Bereich von 3:100 zu 25:100, besonders bevorzugt im Bereich von 5:100 bis 20:100, insbesondere bevorzugt im Bereich von 7:100 bis 19:100 und noch mehr bevorzugt im Bereich von 8:100 bis 18:100 liegt.

Der Anteil von HPA und NPG im Hydrierfeed beträgt erfindungsgemäß mindestens 50 Gew.-%, bevorzugt mindestens 60 Gew.-%, besonders bevorzugt mindestens 65 Gew.-% und insbesondere bevorzugt mindestens 70 Gew.-%, bezogen auf den zugeführten Hydrierfeed.

In einer bevorzugten Ausführungsform kann ein solcher Hydrierfeed erhalten werden, wenn als NPG-enthaltender Strom ein Teilstrom aus dem Austrag des Hydrierreaktors eingesetzt wird und das Gewichtsverhältnis von HPA-enthaltenden Strom zu NPG-enthaltenden Strom im Bereich von 3:100 bis 20:100, besonders bevorzugt im Bereich von 5:100 bis 15:100 liegt.

Die Bestimmung der Konzentrationen an NPG und/oder HPA im HPA-enthaltenden Strom oder im NPG-enthaltenden Strom oder im Hydrierfeed kann mittels dem Fachmann bekannter Methoden erfolgen. Hierzu kann beispielsweise die Zusammensetzung der jeweiligen Ströme bestimmt werden und das Zusammenführen der Ströme so durchgeführt werden, dass die erfindungsgemäße Zusammensetzung des Hydrierfeeds erhalten wird. Die Steuerung der Mengenströme kann beispielsweise über Regelventile oder Dosierpumpen erfolgen. Die Messung der Ströme kann in der Regel mit üblichen Massedurchflussmessern vorgenommen werden.

Erfindungsgemäß wird weiterhin ein oder mehrere pH-Regulatoren zugeführt, um einen pH-Wert von 7,0 bis 9,0 am Ausgang des Hydrierreaktors einzustellen.

Als pH-Regulator wird eine oder mehrere Substanzen eingesetzt ausgewählt aus der Gruppe bestehend aus tertiärem Amin, einer anorganischen Base, einer anorganischen Säure und einer organischen Säure.
Als tertiäre Amine können Amine, wie sie z. B. in DE-A 28 13 201 und DE A 27 02 582 beschrieben sind, eingesetzt werden. Besonders bevorzugt sind Tri-n-alkylamine, insbesondere Triethylamin, Tri-n-propylamin, Tri-n-butylamin und Trimethylamin.
Ganz besonders bevorzugt sind Trimethylamin (TMA), Triethylamin (TEA) und Tri-n-propylamin (TPA), da diese Verbindungen in der Regel einen niedrigeren Siedepunkt als die bevorzugt gebildeten Polymethylole aufweisen und somit die destillative Entfernung aus dem Reaktionsgemisch erleichtert wird.
Insbesondere bevorzugt wird Trimethylamin (TMA) als tertiäres Amin in die Reaktion eingesetzt.
Besonders vorteilhaft wird als tertiäre Amin dasselbe tertiäre Amin eingesetzt, das bereits zuvor in der Aldolierungsstufe als Katalysator verwendet wurde.

Als anorganische Basen werden bevorzugt Carbonate, Hydrogencarbonate und Hydroxide der Alkali- und Erdalkalimetalle eingesetzt, besonders bevorzugt Na₂CO₃, K₂CO₃, CaCO₃, NaHCO₃, KHCO₃, NaOH, KOH und Ca(OH)₂. Anorganische Basen können als Lösung, bevorzugt als wässrige Lösung, eingesetzt werden, bevorzugt in einer Konzentration von 5 bis 50 Gew.-%.

Als anorganische oder organische Säuren können erfindungsgemäß Mineralsäuren wie Schwefelsäure oder Phosphorsäure oder organische Säuren wie Zitronensäure, Essigsäure oder Ethylhexansäure verwendet werden. Bevorzugt wird Essigsäure verwendet.

Die Menge an pH-Regulator, die dem HPA-enthaltenden Strom zugeführt wird, wird so gewählt, dass der Hydrieraustrag, der nach dem Reaktorausgang entnommen wird, einen pH-Wert von 7,0 bis 9,0 aufweist.

In der Regel werden im erfindungsgemäßen Verfahren bis zu 1 Gew.-% (bezogen auf den Hydrierfeed), vorzugsweise bis zu 0,75 Gew.-% und besonders bevorzugt bis zu 0,5 Gew.-% des tertiären Amins zur Einstellung des pH-Wert auf den erfindungsgemäßen Bereich zugesetzt.
Das Amin kann als Reinsubstanz oder als wässrige Lösung eingesetzt werden.
Amine sind besonders vorteilhaft zur pH-Einstellung zu verwenden, da sie mit Ameisensäure thermisch zersetzbare Salze bilden, welche nach der Hydrierung wieder gespalten werden können. Somit kann ein Salzanfall vermieden werden und das tertiäre Amin kann in den Prozess rückgeführt werden.

Werden anorganische Basen, anorganische Säuren oder organische Säuren zur Einstellung des pH-Werts eingesetzt, so können diese als Reinsubstanz oder als Lösung, bevorzugt als wässrige Lösung eingesetzt werden. Besonders bevorzugt beträgt die Konzentration der eingesetzten wässrigen Lösung von 5 bis 50 Gew.-%.
Besonders bevorzugt werden bis zu 3 Gew.-% (bezogen auf den Hydrierfeed) einer 10%igen wässrigen Lösung der Säure bzw. anorganischen Base zur pH-Einstellung zugesetzt

Die Messung des pH-Werts des Hydrieraustrages am Ausgang des Hydrierreaktors erfolgt im Allgemeinen mit den bekannten Techniken, vorzugsweise mit einer Glaselektrode und einem pH-Meter.
So kann die Messung des pH-Wertes beispielsweise online oder durch regelmäßige Probenentnahme erfolgen.
Die Messung des pH-Wertes erfolgt am Ausgang des Hydrierreaktors, wobei üblicherweise als Ausgang ein Bereich verstanden werden soll, der hinter der Katalysatorpackung/schüttung liegt oder hinter der Katalysatorabtrennung liegt. Bevorzugt erfolgt die pH-Messung am oder kurz nach dem Auslass aus dem Hydrierreaktor. Dabei kann die Messung beispielsweise in einem separaten Umpumpkreislauf oder Probennahmekreislauf stattfinden, der sich kurz hinter dem Auslass des Hydrierreaktor befindet. Wenn die Hydrierung in mehreren hintereinander geschalteten Reaktoren durchgeführt wird, beispielsweise in 2 bis 4 Reaktoren, so erfolgt die Messung des pH-Wertes am Ausgang des letzten Hydrierreaktors.

Die Steuerung der Menge an zugeführtem pH-Regulator erfolgt in der Regel über die Messung des pH-Wertes im Hydrieraustrag, die vorzugsweise online erfolgt. Dabei wird im Allgemeinen die Menge an pH-Regulator so reguliert, dass der pH-Wert im Hydrieraustrag im erfindungsgemäßen Bereich liegt. Die Dosierung des pH-Regulators kann beispielsweise mittels üblicher Regelventile und Dosierpumpen erfolgen.

Die Zuführung der pH-Regulatoren kann erfindungsgemäß zum HPA-enthaltenden Strom oder zum NPG-enthaltenden Strom oder zum Hydrierfeed erfolgen.

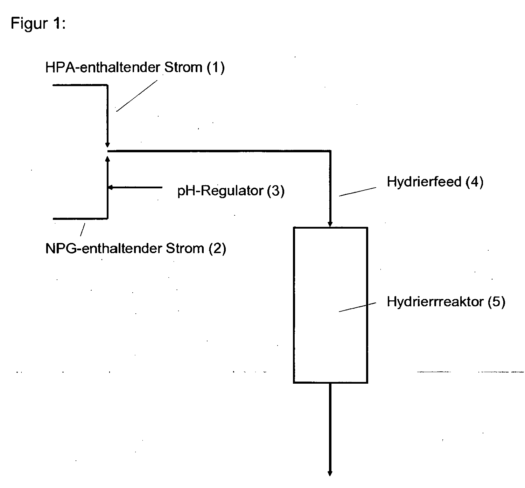

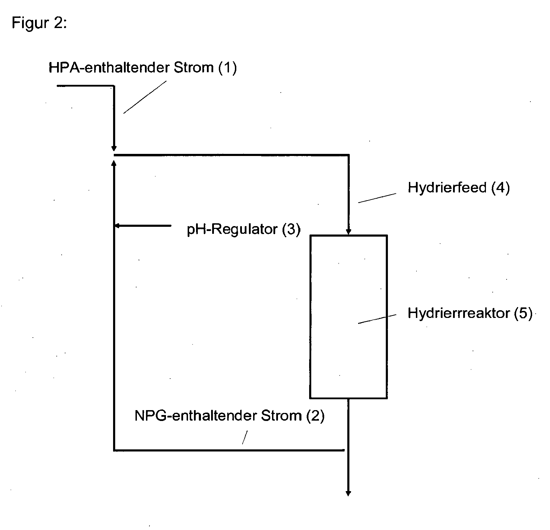

In einer bevorzugten Ausführungsform erfolgt die Zuführung des pH-Regulators zum NPG-enthaltenden Strom.
Eine solche Ausführungsform wird beispielhaft in Figur 1 veranschaulicht. In dieser Ausführungsform wird der HPA-enthaltende Strom (1) mit einem NPG-enthaltenden Strom (2) zu einem Hydrierfeed (4) zusammengeführt. Der Hydrierfeed (4) wird dann in den Hydrierreaktor (5) eingeleitet. Vor der Zusammenführung des NPG-enthaltenden Stroms (2) mit dem HPA-enthaltenden Strom (1) wird dem NPG-enthaltenden Strom ein pH-Regulator (3) zugeführt.
Die zugeführte Menge an pH-Regulator (3) wird wie voranstehend beschrieben so gewählt, dass man am Reaktorausgang einen pH-Wert von 7,0 bis 9,0 einstellt.
Die Temperatur des NPG-enthaltdenden Stroms vor der Zuführung des pH-Regulators liegt vorzugsweise im Bereich von 50 bis 140°C, besonders bevorzugt im Bereich von 70 bis 135°C und insbesondere bevorzugt im Bereich von 85 bis 130°C.
Wie voranstehend beschrieben wird die Menge an zugeführtem NPG so gewählt, dass das Gewichtsverhältnis von HPA zu NPG im Hydrierfeed im Bereich von 1:100 bis 50:100, bevorzugt im Bereich von 3:100 zu 25:100, besonders bevorzugt im Bereich von 5:100 bis 20:100, insbesondere bevorzugt im Bereich von 7:100 bis 19:100 und noch mehr bevorzugt im Bereich von 8:100 bis 18:100 liegt.
In einer ganz besonders bevorzugten Ausführungsform ist der NPG-enthaltende Strom ein Teilaustrag aus dem Hydrierreaktor. Diese Ausführungsform ist in Abbildung 2 beispielhaft veranschaulicht. In dieser Ausführungsform wird der Hydrieraustrag in zwei Ströme aufgespalten, von denen einer als NPG-enthaltender Strom (2) mit dem HPA-enthaltenden Strom (1) zum Hydrierfeed (4) zusammengeführt wird. Der NPG-enthaltende Strom, der ein Teilstrom des Reaktoraustrags ist, wird im Rahmen der vorliegenden Erfindung auch als Umlaufstrom bezeichnet. In dieser bevorzugten Ausführungsform wird der pH-Regulator (3) dem NPG-enthaltenden Umlaufstrom (2) zugeführt. Das Gewichtsverhältnis von HPA-enthaltendem Strom (1) und NPG-enthaltendem Strom (Umlaufstrom) (2) liegt in dieser bevorzugten Ausführungsform bevorzugt im Bereich von 3:100 bis 20:100, besonders bevorzugt im Bereich von 5:100 bis 15:100. Die Temperatur des Umlaufstroms am Ausgang des Hydrierreaktors beträgt in der Regel im Bereich von 50 bis 180°C, vorzugsweise 90 bis 140°C. Der Umlaufstrom kann auf den oben genannten Temperaturbereich gekühlt werden, beispielsweise mittels eines Wärmetauschers.

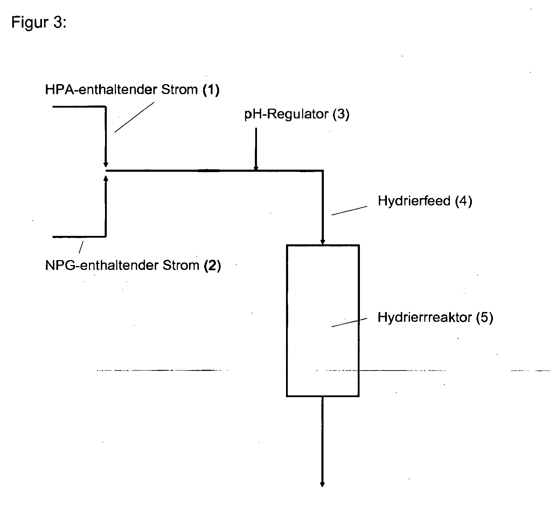

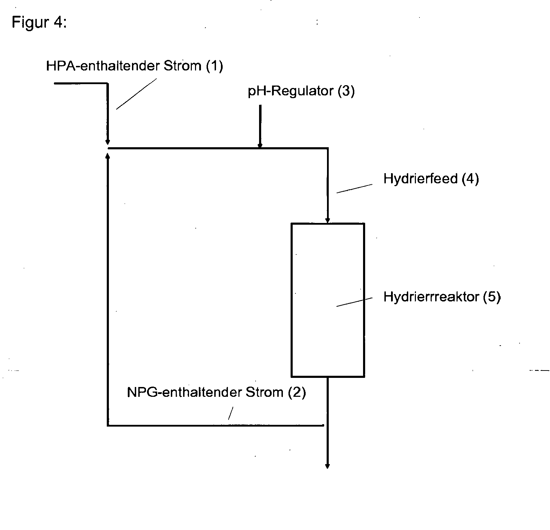

In einer weiteren bevorzugten Ausführungsform erfolgt die Zuführung des pH-Regulators zum Hydrierfeed.
Eine solche Ausführungsform wird beispielsweise in Figur 3 veranschaulicht.
In dieser Ausführungsform wird der HPA-enthaltende Strom (1) zunächst mit dem NPG-enthaltenden Strom (2) zum einem Hydrierfeed (4) zusammengeführt. Vor der Zuführung des Hydrierfeeds (4) in den Hydrierreaktor (5) wird in dieser bevorzugten Ausführungsform dem Hydrierfeed (4) ein pH-Regulator (3) zugeführt.
Die zugeführte Menge an pH-Regulator (3) wird wie voranstehend beschrieben so gewählt, dass man am Reaktorausgang einen pH-Wert von 7,0 bis 9,0 einstellt.
Die Temperatur des Hydrierfeeds vor der Zuführung des pH-Regulators liegt vorzugsweise im Bereich von 50 bis 140°C, besonders bevorzugt im Bereich von 70 bis 135°C und ganz besonders bevorzugt im Bereich von 85 bis 130°C.
Wie voranstehend beschrieben wird die Menge an zugeführtem NPG so gewählt, dass das Gewichtsverhältnis von HPA zu NPG im Hydrierfeed im Bereich von 1:100 bis 50:100, bevorzugt im Bereich von 3:100 zu 25:100, besonders bevorzugt im Bereich von 5:100 bis 20:100, insbesondere bevorzugt im Bereich von 7:100 bis 19:100 und noch mehr bevorzugt im Bereich von 8:100 bis 18:100 liegt.
In einer ganz besonders bevorzugten Ausführungsform ist der NPG-enthaltende Strom ein Teilaustrag aus dem Hydrierreaktor. Diese Ausführungsform ist in Abbildung 4 beispielhaft veranschaulicht. In dieser Ausführungsform wird der Hydrieraustrag in zwei Ströme aufgespalten, von denen einer als NPG-enthaltender Strom (2) mit dem HPA-enthaltenden Strom (1) zum Hydrierfeed (4) zusammengeführt wird. Der NPG-enthaltende Strom, der ein Teilstrom des Reaktoraustrags ist, wird im Rahmen der vorliegenden Erfindung auch als Umlaufstrom bezeichnet. In dieser bevorzugten Ausführungsform wird der pH-Regulator (3) dem Hydrierfeed (4) zugeführt. Das Gewichtsverhältnis von HPA-enthaltendem Strom (1) und NPG-enthaltendem Strom (Umlaufstrom) (2) liegt in dieser bevorzugten Ausführungsform bevorzugt im Bereich von 3:100 bis 20:100, besonders bevorzugt im Bereich von 5:100 bis 15:100. Die Temperatur des Umlaufstrom am Ausgang des Hydrierreaktors beträgt in der Regel im Bereich von 80 bis 180°C, vorzugsweise 90 bis 140°C. Der Umlaufstrom kann auf den oben genannten Temperaturbereich gekühlt werden, beispielsweise mittels eines Wärmetauschers.

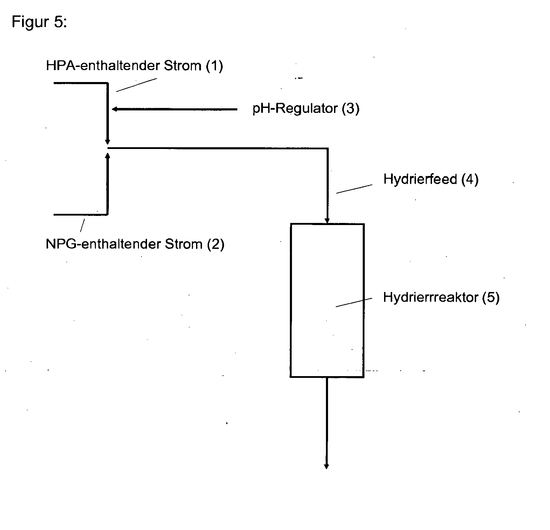

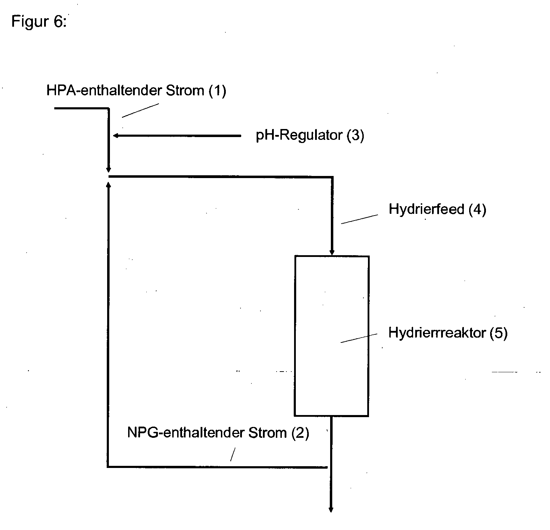

In einer weiteren Ausführungsform dieser Erfindung kann der pH-Regulator auch dem HPA-enthaltendem Strom zugeführt werden.
Eine solche Ausführungsform ist beispielsweise in Figur 5 veranschaulicht. In dieser Ausführungsform wird zum HPA-enthaltenden Strom (1) zunächst der pH-Regulator (3) zugeführt, bevor der HPA-enthaltende Strom (1) mit dem NPG-enthaltenden Strom (2) zum Hydrierfeed (4) zusammengeführt wird.
In dieser Ausführungsform muss darauf geachtet werden - wie nachfolgend beschrieben --, dass die Konzentration des HPA im HPA-enthaltenden Stroms gering ist oder die Temperatur des HPA-enthaltenden Strom bei der Zuführung des pH-Regulators niedrig ist oder die Verweilzeit zwischen der Zuführung des pH-Regulators und der anschließenden Zuführung des NPG-enthaltenden Stroms kurz ist.
Wenn das Gewichtsverhältnis von HPA im HPA-enthaltenden Strom vor der Zuführung des pH-Regulators weniger als 50 Gew.-%, bevorzugt weniger als 40 Gew.-% und besonders bevorzugt weniger als 30 Gew.-% beträgt, dann liegt die Temperatur im HPA-enthaltenden Strom bei der Zuführung des pH-Regulators vorzugsweise im Bereich von 50 bis 100°C, besonders bevorzugt im Bereich von 60 bis 80°C, wobei die Temperatur nicht so niedrig gewählt werden sollte, dass das HPA im HPA-enthaltenden Strom fest wird. Die Verweilzeit zwischen der Zuführung des pH-Regulators und der Zusammenführung des HPA-enthaltenden Stroms mit dem NPG-enthaltenden Strom zum Hydrierfeed kann in diesem Fall mehr als 5 Minuten betragen, vorzugsweise beträgt die Verweilzeit jedoch 1 bis 30 Minuten, besonders bevorzugt 3 bis 15 Minuten. Wenn das Gewichtsverhältnis von HPA im HPA-enthaltenden Strom vor der Zuführung des pH-Regulators mehr als 50 Gew.-% beträgt, dann sollte die Temperatur des HPA-enthaltenden Stroms vor der Zuführung des pH-Regulators vorzugsweise weniger als 75°C, besonders bevorzugt weniger als 70°C betragen, wobei beachtet werden muss, dass die Temperatur nicht so niedrig gewählt wird, dass das HPA im HPA-enthaltende Strom fest wird. Die Verweilzeit zwischen der Zuführung des pH-Regulators und der Zusammenführung des HPA-enthaltenden Stroms mit dem NPG-enthaltenden Strom zum Hydrierfeed kann in diesem Fall mehr als 5 Minuten betragen, vorzugsweise beträgt die Verweilzeit jedoch 1 bis 30 Minuten, besonders bevorzugt 3 bis 15 Minuten. Wenn das Gewichtsverhältnis von HPA im HPA-enthaltenden Strom vor der Zuführung des pH-Regulators mehr als 50 Gew.-% beträgt und die Temperatur des HPA-enthaltenden Stroms vor der Zuführung des pH-Regulatorsmehr mehr als 60°C beträgt, dann sollte die Verweilzeit zwischen der Zuführung des pH-Regulators und der Zusammenführung des HPA-enthaltenden Stroms mit dem NPG-enthaltenden Stroms zum Hydrierfeed weniger als 5 Minuten, besonders bevorzugt weniger als 3 Minuten und ganz besonders bevorzugt weniger als 1 Minute betragen.
Wie voranstehend beschrieben wird die Menge an zugeführtem NPG so gewählt, dass das Gewichtsverhältnis von HPA zu NPG im Hydrierfeed im Bereich von 1:100 bis 50:100, bevorzugt im Bereich von 3:100 zu 25:100, besonders bevorzugt im Bereich von 5:100 bis 20:100, insbesondere bevorzugt im Bereich von 7:100 bis 19:100 und noch mehr bevorzugt im Bereich von 8:100 bis 18:100 liegt.
Die zugeführte Menge an pH-Regulator (3) wird wie voranstehend beschrieben so gewählt, dass man am Reaktorausgang einen pH-Wert von 7,0 bis 9,0 einstellt.
In einer bevorzugten Ausführungsform ist der NPG-enthaltende Strom ein Teilaustrag aus dem Hydrierreaktor. Diese Ausführungsform ist in Abbildung 6 beispielhaft veranschaulicht. In dieser Ausführungsform wird der Reaktionsaustrag in zwei Ströme aufgespalten, von denen einer als NPG-enthaltender Strom (2) mit dem HPA-enthaltenden Strom (1) zum Hydrierfeed (4) zusammengeführt wird. Der NPG-enthaltende Strom, der ein Teilstrom des Reaktoraustrags ist, wird im Rahmen der vorliegenden Erfindung auch als Umlaufstrom bezeichnet. In dieser Ausführungsform wird der pH-Regulator (3) dem HPA-enthaltenden Strom (1) zugeführt. Das Gewichtsverhältnis von HPA-enthaltendem Strom (1) und NPG-enthaltendem Strom (Umlaufstrom) (2) liegt in dieser bevorzugten Ausführungsform bevorzugt im Bereich von 3:100 bis 20:100, besonders bevorzugt im Bereich von 5:100 bis 15:100.
Die Temperatur des Umlaufstroms am Ausgang des Hydrierreaktors beträgt in der Regel im Bereich von 50 bis 180°C, vorzugsweise 90 bis 140°C. Der Umlaufstrom kann auf den oben genannten Temperaturbereich gekühlt werden, beispielsweise mittels eines Wärmetauschers.
Diese Ausführungsformen, in denen die Zuführung des pH-Regulators zum HPA-enthaltenden Strom erfolgt, sind zwar weniger bevorzugt als die oben genannten bevorzugten Ausführungsformen, in denen die Zuführung des pH-Regulator zum NPGenthaltenen Strom oder zum Hydrierfeed erfolgt, ermöglichen aber dieselben technischen Vorteile.

Die Hydrierung von HPA erfolgt in einem oder mehreren Reaktoren mit Wasserstoff in Gegenwart eines Hydrierkatalysators.
Als Katalysatoren können bevorzugt Katalysatoren eingesetzt werden, die bevorzugt mindestens ein Metall der Nebengruppe 8 bis 12 des Periodensystems der Elemente wie Fe, Ru, Os, Co, Rh, Ir, Ni, Pd, Pt, Cu, Ag, Au, Zn, Cd, Hg, bevorzugt Fe, Co, Ni, Cu, Ru, Pd, Pt, besonders bevorzugt Ni und Cu und insbesondere bevorzugt Cu, bevorzugt auf einem üblichen Trägermaterial, besonders bevorzugt auf einem aus den Oxiden des Titans, Zirkoniums, Hafniums, Siliziums und/oder Aluminiums aufweisen. Die Herstellung der erfindungsgemäß verwendbaren Katalysatoren kann nach aus dem Stand der Technik bekannten Verfahren zur Herstellung derartigen Trägerkatalysatoren erfolgen. Bevorzugt verwendet werden können auch Trägerkatalysatoren, die Kupfer auf einem Aluminiumoxid- oder Titandioxid-haltigen Trägermaterial in An- oder Abwesenheit eines oder mehrer der Elemente Magnesium, Barium, Zink oder Chrom aufweist. Derartige Katalysatoren und ihre Herstellung sind aus WO 99/44974 bekannt. Weiterhin sind kupferhaltige Trägerkatalysatoren wie sie z.B. in WO 95/32171 beschrieben sind und die in EP-A 44 444 und DE 19 57 591 offenbarten Katalysatoren für die erfindungsgemäße Hydrierung geeignet.

Die Hydrierung wird kontinuierlich in einem oder mehreren Hydrierreaktoren durchgeführt.
Als Reaktor wird bevorzugt ein mit einer Katalysatorschüttung gefüllten Reaktorrohr eingesetzt, bei dem die Reaktionslösung über die Katalysatorschüttung z.B. in Riesel- oder Sumpffahrweise, wie in DE A 19 41 633 oder DE A 20 40 501 beschrieben, geleitet wird.
Als Reaktor kann aber auch ein kontinuierlich betriebener Rührkesselreaktor eingesetzt werden.
In einer bevorzugten Ausführungsform wird die Hydrierung in einem Hydrierreaktor durchgeführt, der aus zwei oder mehreren hintereinander geschalteten Reaktoren besteht, z.B. in einer Rührkesselkaskade oder in mehreren hintereinandergeschalteten Rohrreaktoren.
Besonders bevorzugt wird die Hydrierung in 2 bis 4, besonders bevorzugt 2, hintereinandergeschalteten Rohrreaktoren durchgeführt, wobei in den einzelnen Reaktoren vor dem letzten Reaktor, die Hydrierreaktion nur bis zu einem Teilumsatz von z.B. 50 bis 98 % durchgeführt wird und erst im letzten Reaktor die Hydrierung vervollständigt wird. In einer weiteren bevorzugten Ausführungsform wird ein Teilstrom des Reaktionsaustrages, gegebenenfalls unter Kühlung, zurückgeführt und wieder in den Reaktor zurückgeführt. Besonders bevorzugt wird der Teilstrom aus dem ersten Hydrierreakor als Umlaufstrom zurückgeführt. Diese Kreislauffahrweise wird bevorzugt mit einem Verhältnis von Zulauf (HPA-enthaltender Strom) zu Kreislauf (NPG-enthaltender Umlaufstrom) von 3:100 bis 20:100 und besonders bevorzugt im Bereich von 5:100 bis 15:100 betrieben.

Die Hydriertemperatur liegt im Allgemeinen zwischen 50 und 180°C, bevorzugt 90 und 140°C. Als Hydrierdruck werden im allgemeinen 10 bis 250 bar, bevorzugt 20 bis 120 bar angewandt.

Die Hydrierung kann unter Zugabe eines inerten Lösungsmittel durchgeführt werden. Als Verdünnungsmittel sind Wasser, cyclische Ether, wie THF oder Dioxan, als auch acyclische Ether einsetzbar, ebenso niedere Alkohole z.B. Methanol, Ethanol oder 2-Ethylhexanol.
Vorzugsweise erfolgt die Hydrierung aber ohne Zugabe von inerten Lösungsmitteln, da wie voranstehend beschrieben eine Zugabe von Lösungsmittel die Konzentration von HPA im Hydrierfeed verringern würde.

Durch das erfindungsgemäße Verfahren ist es möglich Nebenreaktionen bei der Hydrierung zu verringern, die Selektivität der Umsetzung von HPA zu NPG zu erhöhen und die Ausbeute an NPG zu steigern. Bei der Umsetzung von Hydroxypivalinaldehyd zu NPG kann beispielsweise die Bildung von Hydroxypivalinsäure (HPS) reduziert werden.
Mittels des erfindungsgemäßen Verfahrens ist es weiterhin möglich NPG mit hoher Ausbeute über einen langen Zeitraum herzustellen, d.h. dass die Betriebsdauer der eingesetzten Katalysatoren erhöht wird und Wechsel der eingesetzten Katalysatoren weniger oft erforderlich sind. Weiterhin kann die zugegebene Menge der pH-Regulatoren verringert werden, um die Menge der eingesetzten Rohstoffe zu reduzieren.

Die Erfindung wird anhand der folgenden Beispiele erläutert:

### Beispiel 1:

### Herstellung eines HPA-enthaltenden Stroms

1,1 mol Isobutyraldehyd wurde mit 1 mol Formaldehyd in Form einer 49 %igen Lösung und 4 mol.-% Trimethylamin, bezogen auf Isobutyraldehyd, bei 70-75°C 1,5 h lang gerührt. Die Reaktionslösung wurde eingeengt, indem bei Normaldruck Leichtsieder wie beispielsweise Isobutyraldehyd und Amin abdestilliert wurden. Der erhaltene Sumpf bestand aus 70 Gew.-% Hydroxypivalinaldehyd, 25 Gew.-% Wasser und ca. 5 Gew.-% sonstigen organischen Nebenkomponenten.

### Beispiel 2 (Vergleichsversuch):

### Hydrierung des HPA-enthaltenden Stroms aus Beispiel 1

Als HPA-enthaltender Strom wurde das gemäß Beispiel 1 hergestellte Gemisch eingesetzt.
Dieser HPA-enthaltende Strom wurde zunächst in einen Hydrierreaktor geleitet, der in Rieselfahrweise bei 37-40 bar H₂-Druck und bei 100-120°C betrieben wurde. Die Belastung betrug 0,28 kg Lösung/(lKat.*h). Als Katalysator wurde ein Cu/Al₂O₃-Katalysators wie in der EP-A-44444 bzw. WO-A-2007/042456 beschrieben eingesetzt der gemäß der Offenbarung der EP-A-44444 bzw. WO-A-2007/042456 in einem Rohrreaktor bei 190°C durch Überleiten eines Gemisches aus 5 Vol.% Wasserstoff und 95 Vol.% Stickstoff (Gesamtvolumen 50 NI/h) drucklos 24 h aktiviert wurde.

Der Hydrieraustrag wurde in zwei Teilströme aufgeteilt.
Im stationären Betrieb wurde ein Teilstrom als NPG-enthaltender Umlaufstrom mit dem HPA-enthaltenden Strom zusammengeführt.
Das Verhältnis von NPG-enthaltendem Umlaufstrom zum HPA-enthaltendem Strom aus Beispiel 1 betrug ca. 10:1.
Die Zusammensetzung des Umlaufstroms betrug 25 Gew.-% Wasser, 68 Gew.-% NPG, 1,6 Gew.-% HPA, Rest (ca. 6 Gew.-%) sonstige organische Verbindungen.
Der so erhaltende Hydrierfeed wurde anschließend in den Hydrierreaktor eingeleitet.
Die Zusammensetzung des Hydrierfeeds betrug 25 Gew.-% Wasser, 64 Gew.-% NPG, 7 Gew.-% HPA und 4 Gew.-% sonstige organische Verbindungen.
Das Gewichtsverhältnis von HPA zu NPG im Hydrierfeed betrug ca. 1:9.
Der Teil des Hydrieraustrags, der nicht als NPG-enthaltender Umlaufstrom zurückgeführt wurde, wurde über einen zweiten Hydrierreaktor gefahren.
Der zweite Hydrierreaktor wurde bei 37-40 bar und einer Temperatur von 110-130°C betrieben. Als Katalysator wurde derselbe Kataly/sator eingesetzt, wie im ersten Hydrierreaktor.

Im Austrag des zweiten Reaktors wurde ein pH-Wert von ca. 8 eingestellt, indem dem HPA-enthaltenden Strom aus Beispiel 1 Trimethylamin (TMA) (pH-Regulator) zugeführt wurde.

Die Zuführung des pH-Regulators erfolgte vor der Zusammenführung des HPAenthalteden Stroms mit dem NPG-enthaltendem Umlaufstrom.
Die Temperatur des HPA-enthaltenden Stroms aus Beispiel 1 bei der Zuführung des pH-Regulators (TMA) betrug 80°C. Die Verweilzeit zwischen der Zugabe des pH-Regulators und der Zuführung des Umlaufstroms betrug 10 Minuten (> 5 Minuten).

### Beispiel 3 (erfindungsgemäßes Beispiel):

### Hydrierung des HPA-enthaltenden Stroms aus Beispiel 1

Die Hydrierung erfolgte unter denselben Bedingungen wie in Beispiel 2. Zur Einstellung eines pH-Wertes von 8 am Ausgang des zweiten Reaktors wurde der pH-Regulator (TMA) allerdings direkt dem NPG-enthaltenden Umlaufstrom zugeführt, bevor der NPG-enthaltende Strom mit dem HPA-enthaltenden Strom aus Beispiel 1 zusammengeführt wurde. Das Gewichtsverhältnis von HPA zu NPG in dem durch Zusammenführung dieser Ströme erhaltenen Hydrierfeed betrug ca.1:9.
Die Zusammensetzung des Hydrierfeeds betrug 25 Gew.-% Wasser, 64 Gew.-% NPG, 7 Gew.-% HPA und 4 Gew.-% sonstige organische Verbindungen.

Tabelle 1 zeigt Umsätze, Selektivitäten und den HPS-Gehalt nach der Hydrierung für die Beispiele 2 und 3. Die Umsätze wurden mittels Gaschromatographie bestimmt (GC-Methode: Säule: 50 m Chrompack CP-Sil 8 CB, 0.53 mm Durchmesser FD 5,0 Temperaturprogramm: 5 min 50°C isotherm, dann heizen mit 20°C pro min bis 200°C, 17,5 min isotherm halten. Injektor: 250°C Detektor FID: 300°C. Flow 3,8 ml/min Split: 20)

**Tabelle 1:**

| Zugabe von TMA | pH-Wert Zulauf | pH-Wert Hydrieraustrag | HPS nacht Hydrierung [GC-FI%] | TMA Verbrauch g/kg IBa | Temperatur Dosierung [°C] | Umsatz [%] | Selektivität [%] |
|---|---|---|---|---|---|---|---|
| Beispiel 2 | 8,2 | 7,9 | 0,625 | 15,1 | 80 | 99,2 | 97,6 |
| Beispiel 3 | 8,0 | 8,2 | 0,042 | 5,55 | 107 | 99,9 | 98,9 |

## Patentansprüche

1. Verfahren zur Herstellung Neopentylglykol (NPG) durch kontinuierliche Hydrierung von Hydroxypivalinaldehyd (HPA)
mit Wasserstoff,
in der Flüssigphase,
in Gegenwart eines Hydrierkatalysators,
in einem Hydrierreaktor (5),
wobei man einen HPA-enthaltenden Strom (1) mit einem NPG-enthaltenden Strom (2) zu einem Hydrierfeed (4) zusammenführt und man den Hydrierfeed (4) in den Hydrierreaktor (5) einleitet
und man zusätzlich mindenstens einen pH-Regulator (3) ausgewählt aus der Gruppe bestehend aus tertiärem Amin, einer anorganischen Base, einer anorganischen und einer organischen Säure, dem HPA-enthaltenden Strom (1) oder dem NPG-enthaltenden Strom (2) oder dem Hydrierfeed (4) zuführt,
um einen pH-Wert von 7,0 bis 9,0 am Ausgang des Hydrierreaktors einzustellen, **dadurch gekennzeichnet, dass**
das Gewichtsverhältnis von HPA zu NPG im Hydrierfeed (4) im Bereich von 1:100 bis 50:100 liegt und
der Anteil von HPA und NPG im Hydrierfeed (4) mindestens 50 Gew.-% beträgt, bezogen auf den Hydrierfeed,
wobei für den Fall, dass der pH-Regulator (3) dem HPA-enthaltenden Strom (1) zugeführt wird entweder
der HPA-enthaltende Strom (1) weniger als 50 Gew.-% HPA enthält oder
die Verweilzeit zwischen der Zuführung des pH-Regulators (3) und der Zusammenführung des NPG-enthaltenden Stroms (2) mit dem HPA-enthaltenden Strom (1) weniger als 5 Minuten beträgt oder
die Temperatur des HPA-enthaltenden Stroms (1) weniger als 75°C beträgt.

2. Verfahren nach Anspruch 1, **dadurch gekennzeichnet, dass** man den pH-Regulator (3) zum NPG-enthaltenden Strom (2) zuführt.

3. Verfahren nach Anspruch 1, **dadurch gekennzeichnet, dass** man den pH-Regulator (3) zum Hydrierfeed (4) zuführt.

4. Verfahren nach mindestens einem der Ansprüche 1 bis 3, **dadurch gekennzeichnet, dass** der HPA-enthaltende Strom (1) 50 bis 85 Gew.-% HPA, 15 bis 50 Gew.-% Wasser, Rest: sonstige organische Verbindungen enthält.

5. Verfahren nach mindestens einem der Ansprüche 1 bis 4, **dadurch gekennzeichnet, dass** der NPG-enthaltende Strom (2) 50 bis 90 Gew.-% NPG enthält.

6. Verfahren nach mindestens einem der Ansprüche 1 bis 4, **dadurch gekennzeichnet, dass** das Gewichtsverhältnis von HPA zu NPG im Hydrierfeed (4) im Bereich von 3:100 zu 25:100 liegt.

7. Verfahren nach mindestens einem der Ansprüche 1 bis 6, **dadurch gekennzeichnet, dass** der pH-Regulator Trimethylamin ist.

8. Verfahren nach mindestens einem der Ansprüche 1 bis 7, **dadurch gekennzeichnet, dass** der NPG-enthaltende Strom ein Teilstrom aus dem Austrag des Hydrierreaktors ist.

9. Verfahren nach mindestens einem der Ansprüche 1 bis 8, **dadurch gekennzeichnet, dass** die Hydrierung in zwei oder mehreren hintereinander geschalteten Hydrierreaktoren durchgeführt wird.

## Claims

1. A process for preparing neopentyl glycol (NPG) by continuously hydrogenating hydroxypivalaldehyde (HPA)
with hydrogen,
in the liquid phase,
in the presence of a hydrogenation catalyst,
in a hydrogenation reactor (5),
by combining an HPA-comprising stream (1) with an NPG-comprising stream (2) to give a hydrogenation feed (4) and introducing the hydrogenation feed (4) into the hydrogenation reactor (5) and additionally supplying at least one pH regulator (3) selected from the group consisting of tertiary amine, an inorganic base, an inorganic acid and an organic acid to the HPA-comprising stream (1) or the NPG-comprising stream (2) or the hydrogenation feed (4),
in order to establish a pH of 7.0 to 9.0 at the outlet of the hydrogenation reactor,
wherein
the weight ratio of HPA to NPG in the hydrogenation feed (4) is in the range from 1:100 to 50:100 and
the proportion of HPA and NPG in the hydrogenation feed (4) is at least 50% by weight, based on the hydrogenation feed,
and, in the case that the pH regulator (3) is supplied to the HPA-comprising stream (1),
the HPA-comprising stream (1) comprises less than 50% by weight of HPA or
the residence time between the supply of the pH regulator (3) and the combining of the NPG-comprising stream (2) with the HPA-comprising stream (1) is less than 5 minutes or
the temperature of the HPA-comprising stream (1) is less than 75°C.

2. The process according to claim 1, wherein the pH regulator (3) is supplied to the NPG-comprising stream (2).

3. The process according to claim 1, wherein the pH regulator (3) is supplied to the hydrogenation feed (4).

4. The process according to at least one of claims 1 to 3, wherein the HPA-comprising stream (1) comprises 50 to 85% by weight of HPA, 15 to 50% by weight of water, remainder: other organic compounds.

5. The process according to at least one of claims 1 to 4, wherein the NPG-comprising stream (2) comprises 50 to 90% by weight of NPG.

6. The process according to at least one of claims 1 to 4, wherein the weight ratio of HPA to NPG in the hydrogenation feed (4) is in the range from 3:100 to 25:100.

7. The process according to at least one of claims 1 to 6, wherein the pH regulator is trimethylamine.

8. The process according to at least one of claims 1 to 7, wherein the NPG-comprising stream is a substream from the discharge of the hydrogenation reactor.

9. The process according to at least one of claims 1 to 8, wherein the hydrogenation is performed in two or more hydrogenation reactors connected in series.

## Revendications

1. Procédé pour la préparation de néopentylglycol (NPG) par hydrogénation continue d'hydroxypivalinaldéhyde (HPA) avec de l'hydrogène, en phase liquide, en présence d'un catalyseur d'hydrogénation, dans un réacteur d'hydrogénation (5), en réunissant un flux (1) contenant de l'HPA avec un flux (2) contenant du NPG en une alimentation de l'hydrogénation (4) et en introduisant l'alimentation de l'hydrogénation (4) dans le réacteur d'hydrogénation (5) et en introduisant en outre au moins un régulateur de pH (3), choisi dans le groupe constitué par une amine tertiaire, une base inorganique, un acide inorganique et un acide organique, dans le flux (1) contenant de l'HPA ou dans le flux (2) contenant du NPG ou dans l'alimentation de l'hydrogénation (4), pour régler un pH de 7,0 à 9,0 à la sortie du réacteur d'hydrogénation, **caractérisé en ce que** le rapport pondéral d'HPA à NPG dans l'alimentation de l'hydrogénation (4) se situe dans la plage de 1:100 à 50:100 et la proportion d'HPA et de NPG dans l'alimentation de l'hydrogénation (4) est d'au moins 50% en poids, par rapport à l'alimentation de l'hydrogénation,
où, dans le cas où le régulateur de pH (3) est introduit dans le flux (1) contenant de l'HPA, soit le flux (1) contenant de l'HPA contient moins de 50% en poids d'HPA, soit la durée de séjour entre l'alimentation du régulateur de pH (3) et la réunion du flux (2) contenant du NPG avec le flux (1) contenant de l'HPA est inférieure à 5 minutes, soit la température du flux (1) contenant de l'HPA est inférieure à 75°C.

2. Procédé selon la revendication 1, **caractérisé en ce qu'**on introduit le régulateur de pH (3) dans le flux (2) contenant du NPG.

3. Procédé selon la revendication 1, **caractérisé en ce qu'**on introduit le régulateur de pH (3) dans l'alimentation de l'hydrogénation (4).

4. Procédé selon au moins l'une quelconque des revendications 1 à 3, **caractérisé en ce que** le flux (1) contenant de l'HPA contient 50 à 85% en poids d'HPA, 15 à 50% en poids d'eau, le reste étant d'autres composés organiques.

5. Procédé selon au moins l'une quelconque des revendications 1 à 4, **caractérisé en ce que** le flux (2) contenant du NPG contient 50 à 90% en poids de NPG.

6. Procédé selon au moins l'une quelconque des revendications 1 à 4, **caractérisé en ce que** le rapport pondéral d'HPA à NPG dans l'alimentation de l'hydrogénation (4) se situe dans la plage de 3:100 à 25:100.

7. Procédé selon au moins l'une quelconque des revendications 1 à 6, **caractérisé en ce que** le régulateur de pH est la triméthylamine.

8. Procédé selon au moins l'une quelconque des revendications 1 à 7, **caractérisé en ce que** le flux contenant du NPG est un flux partiel du produit évacué du réacteur d'hydrogénation.

9. Procédé selon au moins l'une quelconque des revendications 1 à 8, **caractérisé en ce que** l'hydrogénation est réalisée dans deux réacteurs d'hydrogénation ou plus, disposés les uns derrière les autres.
